# EUROPEAN PATENT SPECIFICATION

(11) **EP 3 654 928 B1**
(45) Date of publication and mention of the grant of the patent: **28.10.2026**
(21) Application number: 18750035.0
(22) Date of filing: 19.07.2018
(51) Int. Cl.: A61K 8/27, A61K 8/49, A61Q 11/00, C07D 307/91, A61P 1/02, A61P 31/02

(54) **ZINC:USNIC ACID COMPLEXES, ORAL CARE COMPOSITIONS AND METHODS OF USE**
ZINK-USNINSÄURE-KOMPLEXE, MUNDPFLEGEZUSAMMENSETZUNGEN UND ANWENDUNGSVERFAHREN
COMPLEXES DE ZINC : ACIDE USNIQUE, COMPOSITIONS DE SOIN BUCCAL ET PROCÉDÉS D'UTILISATION

(30) Priority: 19.07.2017 US 201762534446 P
(43) Date of publication of application: 27.05.2020
(73) Proprietor: Colgate-Palmolive Company, New York, NY 10022 (US)
(72) Inventor: JARACZ, Stanislav, Somerset, New Jersey 08873 (US); PAN, Long, Somerset, New Jersey 08873 (US); SAMBANTHAMOORTHY, Karthik, Bridgewater, New Jersey 08807 (US)
(74) Representative: Sonnenhauser, Thomas Martin
(86) International application number: PCT/US2018/042790
(87) International publication number: WO 2019/018588

(56) References cited:
- EP-A1- 0 256 566
- DATABASE WPI Week 201649, Derwent World Patents Index; AN 2016-210930, XP002784770
- DATABASE WPI Week 201727, Derwent World Patents Index; AN 2017-153613, XP002784771

## Description

### FIELD

The presently disclosed embodiments or implementations relate to oral care compositions comprising zinc:usnate complexes as well as to methods of using and of making these compositions.

### BACKGROUND

A variety of human ailments owe their origin to pathogenic microorganisms, which include bacteria, virus and fungi. The presence of such pathogenic microorganisms lead to septicaemia, serious infections of upper and lower respiratory tract, CNS, meningitis, intra-abdominal tissue including peritoneum, genito-urinary tract, skin, and soft tissue, and a variety of other infections like systemic mycosis, candidiasis including infections caused by dermatophytes. During the last 100 years, significant progress has been made to combat the diseases caused by such a large family of microbes with innumerable therapeutic agents of diverse chemical and biological nature that have become available as a short and long term cure. Such antimicrobials include aminoglycosides, penicillins, cephalosporins, macrolides, glycopeptides, fluoroquinolones, tetracycline, first and second line anti-TB drugs, anti-leprosy, anti-virals, polyene, triazole and imidazole anti-fungals, combinations like pyrimidine derivatives and trimethoprim and sulphamethoxizole.

Solubility and efficacy of antimicrobial entities plays a significant role in delivery and formulation options. For example, oral delivery with poor aqueous solubility is challenging and often results in poor bioavailability, thus it is desirable for antimicrobials to have water solubility. Having antimicrobial entities that maintain water solubility while also maintaining high efficacy levels are advantageous.

Current antimicrobial products do not adequately address water solubility and efficacy attributes. Accordingly, there is a need for such antimicrobial entities to treat and/or prevent progression of microorganism induced ailments. EP 0 256 566 A1 discloses compositions containing usnic acid or its derivatives useful for the therapeutical control of caries, particularly for the preventive treatment and for the therapy of the cariogenic dental plaque. CN 105411871 A discloses a compound medicine toothpaste comprising an abradant, a wetting agent and deionized water that are taken as the main components, and a composition of tranexamic acid, organic acid zinc, usnic acid and/or usnic acid salt that is added.

### SUMMARY

It has been surprisingly and unexpectedly found that complexes of zinc and usnate having one mole of zinc to two moles of usnate may be produced. These complexes show unexpected physical-chemical attributes, such as an increase in solubility while maintaining antibacterial activity. Such activity may be useful for use in oral care compositions.

In certain embodiments, an oral care composition is disclosed. The oral care composition is comprising a Zn:usnic complex wherein said Zn:usnic acid complex has a 1:2 zinc to usnate molar ratio, wherein the oral care composition is selected from the group consisting of: a toothpaste, a dentifrice, a mouthwash, a mouth rinse, a topical oral gel, and a denture cleanser as defined in the claims. In certain embodiments, the oral care composition further comprises one or more agents selected from diluents, bicarbonate salts, pH modifying agents, surfactants, foam modulators, additional thickening agents, humectants, sweeteners, flavorants, pigments, antibacterial agents, anticaries agents, fluoride ion sources, anticalculus or tartar control agents, and mixtures thereof.

In certain embodiments, oral care compositions as defined in the claims are for use in a method to improve oral health comprising applying an effective amount of said oral composition to the oral cavity of a subject in need thereof are disclosed. The method may include applying an effective amount of any of the oral compositions described herein to the oral cavity of a subject in need thereof. In certain embodiments, the oral health may be selected from one or more of the following; reduce or inhibit formation of dental caries; reduce, repair or inhibit early enamel lesions; reduce or inhibit demineralization and promote remineralization of the teeth; reduce hypersensitivity of the teeth; reduce or inhibit gingivitis; promote healing of sores or cuts in the mouth; reduce levels of acid producing bacteria; to increase relative levels of arginolytic bacteria; inhibit microbial biofilm formation in the oral cavity; raise and/or maintain plaque pH at levels of at least pH 5.5 following sugar challenge; reduce plaque accumulation; treat, relieve or reduce dry mouth; whiten teeth; enhance systemic health, including cardiovascular health; reduce erosion of the teeth; immunize the teeth against cariogenic bacteria and their effects; clean the teeth and oral cavity; reduce inflammation; and increase anti-oxidant levels.

In further embodiments, a method of preparing a Zn:usnate complex having a 1:2 zinc to usnate molar ratio is disclosed as further defined in the claims. In certain aspects, ZnO is used as a source of Zn for the reaction. In certain embodiments, the complex is prepared at a temperature greater than or equal to about 20°C to less than or equal to about 80°C.

In certain embodiments, preparation of the complex comprises the steps of: mixing a zinc source and usnic acid; adding ethanol; mixing the solution at about 80° C; and optionally isolating said complex. In certain embodiments, preparation of the complex comprises the steps of: mixing a zinc source and acetic acid in water; optionally sonicating the mixture; in a separate container, mixing sodium hydroxide (NaOH) and ethanol; mixing the NaOH and ethanol mixture with usnic acid; mixing the solution containing the zinc source and acetic acid in water with the solution having NaOH, ethanol and usnic acid; and optionally isolating said complex. In certain embodiments, preparation of the complex comprises the steps of: mixing a Zn source, usnic acid and ethanol; adding acetic acid; and optionally isolating said complex.

In certain embodiments, the Zn source is selected from zinc acetate, zinc oxide, zinc chloride, zinc lactate, zinc citrate, or zinc nitrate. In certain embodiments, the Zn source is zinc acetate. In certain embodiments, the Zn source is ZnO.

A composition can be obtained or obtainable by combining the ingredients as set forth in any of the preceding compositions and methods.

### BRIEF DESCRIPTION OF THE DRAWINGS

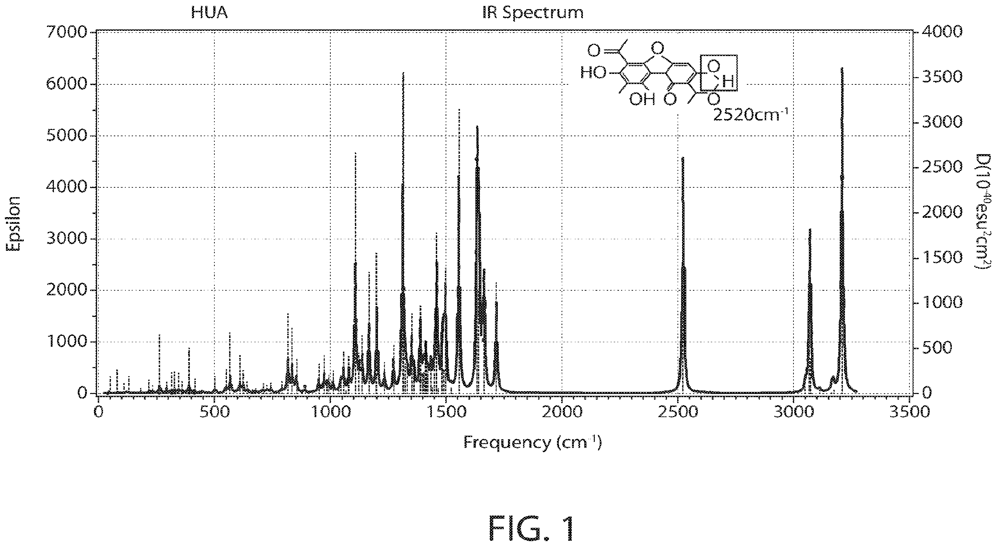

FIG. 1 is a FTIR spectrum of usnic acid.

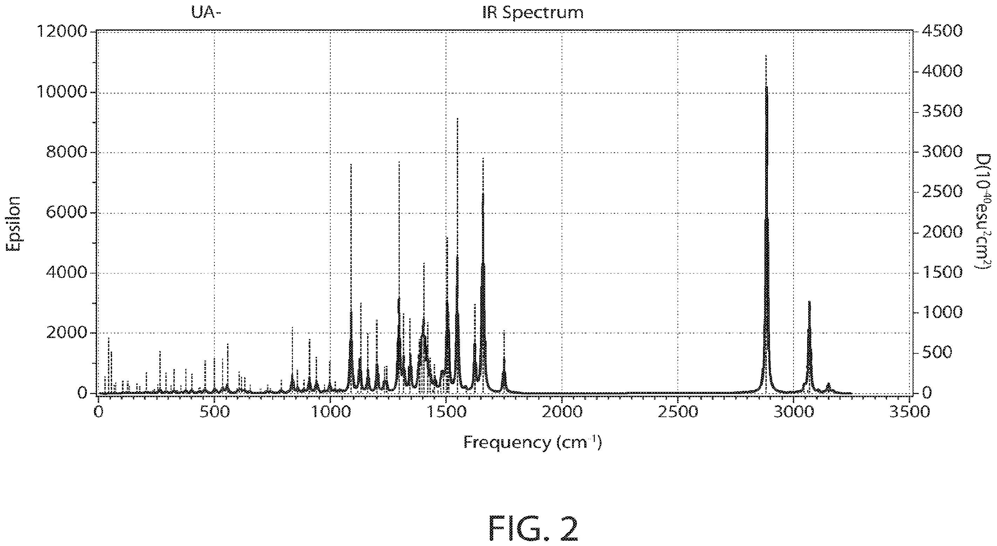

FIG. 2 is a FTIR spectrum of deprotonated usnic acid.

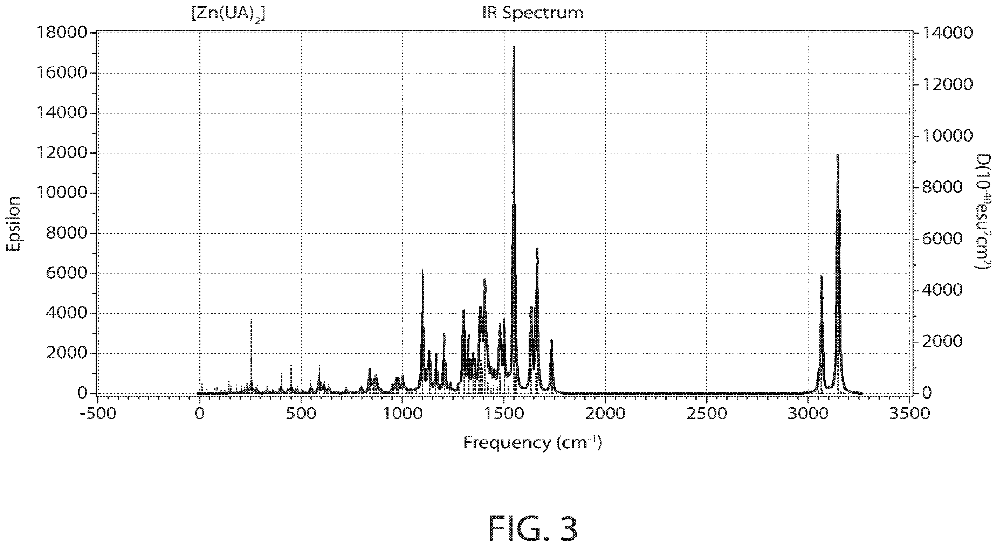

FIG. 3 is a FTIR spectrum of the zinc:usnate complex.
FIG. 4 is a graph depicting percentage of viable bacteria left post treatment with different concentrations of zinc:usnate complex. The % Viability values represent treated samples compared to untreated samples.

### DETAILED DESCRIPTION

The following description of various typical aspect(s) is merely exemplary in nature and is in no way intended to limit the disclosure, its application, or uses.

As used herein, the words "preferred" and "preferably" refer to embodiments that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

As used throughout, ranges are used as shorthand for describing each and every value that is within the range. Any value within the range can be selected as the terminus of the range. Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral. It should further be appreciated that when a numerical range is disclosed herein, any numerical value falling within the range is also specifically disclosed.

Unless stated otherwise, all percentages of composition components given in this specification are by weight based on a total composition or formulation weight of 100%.

As used herein, the term "oral composition" means the total composition that is delivered to the oral surfaces. The composition is further defined as a product which, during the normal course of usage, is not for the purposes of systemic administration of particular therapeutic agents, intentionally swallowed but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, dental strips, beads, varnish, toothpowder and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively, the oral composition may be dual phase dispensed from a separated compartment dispenser.

The term "mouthrinse," as used herein, may refer to oral compositions that are substantially liquid in character, such as a mouth wash, spray, or rinse. In such a preparation the orally acceptable carrier typically has an aqueous phase comprising water or a water and alcohol mixture. Further, in various embodiments, the oral carrier includes a humectant and surfactant as described below. Generally, the weight ratio of water to alcohol is in the range of in an amount of 1:1 to 20:1, preferably 3:1 to 10:1 and more preferably 4:1 to 6:1. The total amount of water-alcohol mixture in this type of preparation is typically in an amount of 70 to 99.9% of the preparation. In various embodiments, the alcohol is typically ethanol or isopropanol.

The term "effective amount" as used herein means that the amount is of sufficient quantity to achieve the intended purpose, such as, for example, treat and/or prevent progression of microorganism induced ailments.

The terms "1:2 Zn:usnate", "2:1 usnate:Zn complex", "zinc:usnate", "Zn:usnate complex" and "zinc:usnate complex" are used interchangeably and refer, unless specified otherwise, to any one or more of the complexes disclosed herein.

Unless otherwise specifically identified, the ingredients for use in the compositions and formulations disclosed herein are preferably cosmetically acceptable ingredients. By "cosmetically acceptable" is meant suitable for use in a formulation for topical application to human skin. A cosmetically acceptable excipient, for example, is an excipient that is suitable for external application in the amounts and concentrations contemplated in the formulations of the present disclosure, and includes, for example, excipients that are "Generally Recognized as Safe" (GRAS) by the United States Food and Drug Administration.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that embodiments of the disclosure extend to any products of the combination of the listed ingredients.

Compositions comprising Zn:usnate complex(es) are disclosed, wherein the Zn:usnate complex has a 1:2 zinc to usnate molar ratio. Such complexes provide unique features, such as enhanced antimicrobial activities, useful in oral care applications. In certain embodiments, the zinc:usnate complex is present at about 7 ppm to about 10 ppm of the final oral care product. In certain embodiments, the complex is present at about 7 ppm to about 100 ppm of the final oral care product. In certain embodiments, the complex is present at about 7 ppm to about 500 ppm of the final oral care product. In certain embodiments, the complex is present at about 7 ppm to about 1000 ppm of the final oral care product.

Methods of preparing a Zn:usnate complex having a 1:2 zinc to usnate molar ratio are disclosed. In certain preferred embodiments, the zinc and usnic acid source are combined in an alcohol solution. In certain embodiments, the zinc and usnic acid source are combined in methanol, ethanol, isopropanol, butanol, or the like.

In certain embodiments, the complex is synthesized at a temperature of from about 20°C to about 80°C. In certain embodiments, the complex is synthesized at a temperature of from about 25°C to about 75°C. In certain embodiments, the complex is synthesized at a temperature of from about 30°C to about 70°C. In certain embodiments, the complex is synthesized at a temperature of from about 35°C to about 65°C. In certain embodiments, the complex is synthesized at a temperature of from about 20°C to about 40°C. In certain embodiments, the complex is synthesized at a temperature of from about 40°C to about 60°C. In certain embodiments, the complex is synthesized at a temperature of from about 60°C to about 80°C. In certain embodiments, the complex is synthesized at a temperature of from about 20°C to about 25°C.

In certain embodiments, the complex is synthesized at a temperature of from about 20°C to about 80°C and in an alcohol solution. In certain embodiments, the complex is synthesized at a temperature of from about 25°C to about 75°C and in an alcohol solution. In certain embodiments, the complex is synthesized at a temperature of from about 30°C to about 70°C and in an alcohol solution. In certain embodiments, the complex is synthesized at a temperature of from about 25°C to 75°C and in an alcohol solution selected from methanol, ethanol, isopropanol, butanol, or the like. In certain embodiments, the complex is synthesized at a temperature of from about 30°C to about 70°C and in an alcohol solution selected from methanol, ethanol, isopropanol, butanol, or the like.

In certain embodiments, the complex is synthesized using one molar equivalent of zinc oxide to usnic acid of 1 to 2.5, or 1:1-2.5 of ZnO to usnic acid. In certain embodiments, the molar equivalent of zinc oxide to usnic acid is 1:1.5 to 1:2.5. In certain embodiments, the molar equivalent of zinc oxide to usnic acid is 1:2 to 1:2.5.

In certain embodiments, the complex is prepared using one molar equivalent of an acid to zinc oxide of 1.8 to 4.0, or 1:1.8-4.0 of acid to ZnO. In certain embodiments, the molar equivalent of an acid to zinc oxide is 1:2.0 to 3.0. In certain embodiments, the acid is selected from a carboxylic acid. In certain embodiments, the acid is selected from formic acid, lactic acid, propionic acid, butyric acid and acetic acid. In certain embodiments, the acid is a non-carboxylic acid. In certain embodiments, the acid is selected from hydrochloric acid, sulfuric acid, nitric acid, phosphoric acid, perchloric acid and hydrobromic acid. In certain embodiments, the acid is acetic acid.

In certain embodiments, a method of preparing the 1:2 zinc:usnate complex is disclosed. In certain embodiments, the complex is prepared at a temperature of from about 20°C to about 80°C. In certain embodiments, preparation of the complex comprises the steps of: contacting or mixing a zinc source and usnic acid; adding ethanol; mixing the solution at about 80°C; and optionally isolating said complex. In further embodiments, preparation of the complex comprises the steps of: mixing a zinc source and acetic acid in water; optionally sonicating the mixture; in a separate container, mixing NaOH and ethanol; mixing the NaOH and ethanol mixture with usnic acid; mixing the solution containing the zinc source and acetic acid in water with the solution having NaOH, ethanol and usnic acid; and optionally isolating said complex. In further embodiments, preparation of the complex comprises the steps of: mixing a Zn source, usnic acid and ethanol; adding acetic acid; and optionally isolating said complex.

A composition comprising the 1:2 zinc:usnate complex for use in reducing and/or inhibiting acid erosion of the enamel, reducing or inhibiting gum recession, controlling microbial growth, cleaning the teeth, reducing bacterially-generated biofilm and plaque, reducing gingivitis, inhibiting tooth decay and formation of cavities, and/or reducing dentinal hypersensitivity is disclosed. A composition comprising the 1:2 zinc:usnate complex for use in controlling microbial growth is disclosed. A composition comprising the 1:2 zinc:usnate complex for use in treating and/or preventing progression of microorganism induced ailments.

In certain embodiments, zinc:usnate complexes are included in oral care compositions. In some embodiments, the oral care composition may be selected from the group selected from a toothpaste or a dentifrice, a mouthwash or a mouth rinse, a topical oral gel and a denture cleanser. In some embodiments, the oral care composition may be toothpaste or a dentifrice. In some embodiments, the oral care composition may be a mouthwash or a mouth rinse. In some embodiments, the oral care composition may be a topical oral gel and a denture cleanser.

In further embodiments, oral care compositions as defined in the claims for use in a method to improve oral health are disclosed. The method comprises applying an effective amount of an oral composition described herein to the oral cavity of a subject in need thereof. In certain embodiments, the method includes use of a 1:2 zinc:usnate complex composition selected from the group consisting of a toothpaste or a dentifrice, a mouthwash or a mouth rinse, a topical oral gel and a denture cleanser.

Oral care compositions as defined in the claims for use in methods to reduce and inhibit acid erosion of the enamel, reduce or inhibit gum recession, control microbial growth, clean the teeth, reduce bacterially-generated biofilm and plaque, reduce gingivitis, inhibit tooth decay and formation of cavities, and reduce dentinal hypersensitivity are disclosed. The method may comprise applying an effective amount of any composition disclosed herein to the teeth.

For example, methods to reduce and inhibit acid erosion of the enamel, reduce or inhibit gum recession, control microbial growth, clean the teeth, reduce bacterially-generated biofilm and plaque, reduce gingivitis, inhibit tooth decay and formation of cavities, and reduce dentinal hypersensitivity, may comprise applying an effective amount of a composition disclosed herein to the oral cavity, and may further comprise rinsing with sufficient water or an aqueous solution.

Some embodiments further comprise an effective amount of a fluoride ion source within the composition.

In other embodiments, an orally acceptable base comprises ingredients selected from one or more of buffering agents, humectants, surfactants, thickeners, breath fresheners, flavoring, fragrance, coloring, antibacterial agents, whitening agents, agents that interfere with or prevent bacterial attachment, calcium sources, phosphate sources, orally acceptable potassium salts, and anionic polymers.

Some embodiments provide a mouthwash for use in reducing or inhibiting acid erosion of the enamel, reducing or inhibiting gum recession, controlling microbial growth, cleaning the teeth, reducing bacterially-generated biofilm and plaque, reducing gingivitis, inhibiting tooth decay and formation of cavities, and/or reducing dentinal hypersensitivity.

The 1:2 zinc:usnate complex can be for use in the manufacture of a mouthwash. The claimed oral care compositions can be used in a method of treating or reducing dental enamel erosion cleaning the teeth, reducing or inhibiting gum recession, reducing bacterially-generated biofilm and plaque, reducing gingivitis, inhibiting tooth decay and formation of cavities, and/or reducing dentinal hypersensitivity comprising applying a mouthwash as described herein. Other embodiments provide methods further comprising the step of rinsing with sufficient water or aqueous solution.

A method of making an oral care composition is disclosed. The method may comprise combining usnic acid or usnate and a zinc source in a liquid medium, optionally isolating the complex thus formed in solid form, and combining the zinc:usnate complex with an oral care composition. The oral care composition can be a toothpaste. The oral care composition can be a mouthwash base.

Disclosed herein are further methods to (i) reduce hypersensitivity of the teeth, (ii) reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; (xv) reduce tartar build-up, and/or (xvi) promote systemic health, including cardiovascular health, e.g., by reducing potential for systemic infection via the oral tissues, comprising applying any of said compositions described herein to the oral cavity of a person in need thereof, e.g., one or more times per day. The oral care compositions can be used to reduce or inhibit gum recession. The oral care compositions can be used in methods to control microbial growth. The oral care compositions can be used in methods to reduce bacterially-generated biofilm, malodor and/or plaque. Disclosed here are also compositions for use in any of the aforementioned methods.

"Actives," means compounds that, when applied to a target tissue, provide a benefit or improvement to the target tissue. The actives can be delivered in the form of any oral care formulations, for example a toothpaste, transparent paste, gel, mouthwash, powder, cream, strip, spray, gum, or any other known in the art.

The mixture may be prepared and immediately transferred into a retaining tray, such as those used in holding whitening gels, and the person can wear the tray for the effective period of time. The teeth that are in contact with the mixture will be treated. For use with retaining tray, the mixture can be in the form of a low-viscosity liquid or a gel. In certain embodiments, the complex is formulated in a composition comprising CARBOPOL^{®} polymer, glycerin and water.

A stock solution, or a mixture of the stock solution with water, may be applied to the teeth in a gel formulation, e.g., wherein the gel can stay on the tooth for an extended period of time for effective treatment.

The composition may be a viscous liquid, preferably a gel, which maintains its consistency during storage enabling the product to be painted on the tooth surface with a soft applicator pen or brush. Some methods may be utilizing an applicator to deliver the composition, wherein the applicator is a pen and the pen is stored within an oral care implement. The pen may be removed from the oral care implement prior to application of the composition to the tooth. The composition may be applied to the tooth after brushing. The composition may be applied to the tooth after brushing with the oral care implement.

The zinc ion source for complex synthesis may be from any source that provides Zn²⁺ ions efficiently, for example zinc oxide, zinc acetate, zinc chloride, zinc lactate, tetrabasic zinc chloride, zinc carbonate, zinc nitrate, zinc citrate, zinc bis lysinate, and zinc phosphate. Zinc oxide is a white powder, insoluble in water. Tetrabasic zinc chloride (TBZC) or zinc chloride hydroxide monohydrate is a zinc hydroxy compound with the formula Zn₅(OH)₈Cl₂·H₂O, also referred to as basic zinc chloride, zinc hydroxychloride, or zinc oxychloride. It is a colorless crystalline solid insoluble in water. Both of these materials may be solubilized in water in the presence of usnic acid or usnate and heat, thus providing a source of zinc ions. In certain preferred embodiments, the Zn source is selected from zinc acetate, zinc oxide, zinc chloride, zinc lactate, zinc citrate, or zinc nitrate.

In certain embodiments, the amount of zinc in the composition is 0.005 to 30% by weight of the composition. In certain embodiments, precursors, e.g., zinc sources and usnic acid, are present in amounts such that when combined into the zinc:usnate complex, the complex would be present in an amount of 0.005 to 10% by weight of the composition. In either of these embodiments, the amount of the 1:2 zinc:usnate complex can be varied for the desired purpose, such as a dentifrice or a mouthwash. In other embodiments, the amount of the 1:2 zinc:usnate complex is at least 0.1, at least 0.2, at least 0.3, at least 0.4, at least 0.5, at least 1, at least 2, at least 3, or at least 4 up to 10% by weight of the composition. In other embodiments, the amount of the 1:2 zinc:usnate complex is less than 9, less than 8, less than 7, less than 6, less than 5, less than 4, less than 3, less than 2, less than 1, less than 0.5 to 0.005% by weight of the composition. In other embodiments, the amounts are 0.05 to 5%, 0.05 to 4%, 0.05 to 3%, 0.05 to 2%, 0.1 to 5%, 0.1 to 4%, 0.1 to 3%, 0.1 to 2%, 0.5 to 5%, 0.5 to 4%, 0.5 to 3%, or 0.5 to 2% by weight of the composition.

In certain embodiments, the composition is anhydrous. By anhydrous, there is less than 5% by weight water, optionally less than 4, less than 3, less than 2, less than 1, less than 0.5, less than 0.1 down to 0% by weight water.

In certain embodiments, oral care compositions having zinc:usnate complexes further comprise one or more agents selected from diluents, bicarbonate salts, pH modifying agents, surfactants, foam modulators, additional thickening agents, humectants, sweeteners, flavorants, pigments, antibacterial agents, anticaries agents, fluoride ion sources, anticalculus or tartar control agents, and mixtures thereof.

The oral composition may optionally include other materials, such as for example, cleaning agents, flavouring agents, sweetening agents, adhesion agents, surfactants, foam modulators, abrasives, pH modifying agents, humectants, moisturizers, mouth feel agents, colorants, abrasives, preservatives, fluoride ion source, saliva stimulating agents, emollients, viscosity modifiers, diluents, emulsifiers, nutrients and combinations thereof. Various components that may be added to the oral composition include, for example, a sweetening agent such as saccharin, or sodium saccharin, alcohols such as ethanol, fluoride ion sources such as sodium fluoride, as well as glycerine, sorbitol, polyethylene glycols. Poloxamer polymers such as POLOXOMER^{®} 407, PLURONIC^{®} F108, (both available from BASF Corporation), alkyl polyglycoside (APG), polysorbate, PEG40, castor oil, menthol, and the like. It is understood that while general attributes of each of the above categories of materials may differ, there may be some common attributes and any given material may serve multiple purposes within two or more of such categories of materials. Preferably, such carrier materials are selected for compatibility with the active ingredients found in magnolia extract or synthetic analogues thereof, as well as with other ingredients of the composition.

Flavorants among those useful herein include any material or mixture of materials operable to enhance the taste of the composition. Any orally acceptable natural or synthetic flavorant can be used, such as flavoring oils, flavoring aldehydes, esters, alcohols, similar materials, and combinations thereof. Flavorants include vanillin, sage, marjoram, parsley oil, spearmint oil, cinnamon oil, oil of wintergreen (methylsalicylate) peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants, and mixtures thereof. Also encompassed within flavorants herein are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, [alpha]-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methane glycerol acetal (MGA) and mixtures thereof. One or more flavorants are optionally present in a total amount of 0.01% to 5%, optionally in various embodiments from 0.05 to 2%, from 0.1% to 2.5%, and from 0.1 to 0.5%.

Sweetening agents among those useful herein include dextrose, polydextrose, sucrose, maltose, dextrin, dried invert sugar, mannose, xylose, ribose, fructose, levulose galactose, corn syrup, partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones, and mixtures thereof.

Mouth-feel agents include materials imparting a desirable texture or other feeling during use of the composition of the present disclosure.

Colorants among those useful herein include pigments, dyes, lakes and agents imparting a particular luster or reflectivity such as pearling agents. In various embodiments, colorants are operable to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including FD&C dyes and pigments, talc, mica, magnesium carbonate, calcium carbonate, magnesium silicate, magnesium aluminum silicate, silica, titanium dioxide, zinc oxide, red, yellow, brown and black iron oxides, ferric ammonium ferrocyanide, manganese violet, ultramarine, titaniated mica, bismuth oxychloride, and mixtures thereof. One or more colorants are optionally present in a total amount of 0.001% to 20%, for example 0.01% to 10% or 0.1% to 5%.

### Active Agents:

The compositions may comprise various agents which are active to protect and enhance the strength and integrity of the enamel and tooth structure and/or to reduce bacteria and associated tooth decay and/or gum disease, including or in addition to the zinc-amino acid-halide complexes. Effective concentration of the active ingredients used herein will depend on the particular agent and the delivery system used. It is understood that a toothpaste for example will typically be diluted with water upon use, while a mouth rinse typically will not be. Thus, an effective concentration of active in a toothpaste will ordinarily be 5-15× higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Arginine, where present, may be present at levels from, e.g., about 0.1 to about 20 weight % (expressed as weight of free base), e.g., about 1 to about 10 weight % for a consumer toothpaste or about 7 to about 20 weight % for a professional or prescription treatment product. Fluoride where present may be present at levels of, e.g., about 25 to about 25,000 ppm, for example about 750 to about 2,000 ppm for a consumer toothpaste, or about 2,000 to about 25,000 ppm for a professional or prescription treatment product. Levels of antibacterial agents will vary similarly, with levels used in toothpaste being e.g., about 5 to about 15 times greater than used in mouthrinse. For example, a triclosan toothpaste may contain about 0.3 weight % triclosan.

### Fluoride Ion Source:

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions at a level of about 0.01 weight % to about 10 weight % in one embodiment or about 0.03 weight % to about 5 weight %, and in another embodiment about 0.1 weight % to about 1 weight % by weight of the composition in another embodiment. Weights of fluoride salts to provide the appropriate level of fluoride ion will obviously vary based on the weight of the counterion in the salt.

### Foaming Agents:

The oral care compositions may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component. Polyoxyethylene is also commonly known as polyethylene glycol ("PEG") or polyethylene oxide. The polyoxyethylenes suitable may have a molecular weight of about 200,000 to about 7,000,000. In one embodiment, the molecular weight will be about 600,000 to about 2,000,000 and in another embodiment about 800,000 to about 1,000,000. POLYOX^{®} is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions. Where present, the amount of foaming agent in the oral care composition (i.e., a single dose) is about 0.01 to about 0.9% by weight, about 0.05 to about 0.5% by weight, and in another embodiment about 0.1 to about 0.2% by weight.

### Tartar Control Agents:

In various embodiments, the compositions comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The compositions may comprise phosphate salts. In particular embodiments, these salts are alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P1-6 phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of ca. 3-4% of the sodium phosphate dibasic and ca. 0.2-1% of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

### Polymers:

The oral care compositions may also include additional polymers to adjust the viscosity of the formulation or enhance the solubility of other ingredients. Such additional polymers include polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as GANTREZ AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of about 0.5% to about 5.0% by weight of the total composition are used.

The compositions may include an anionic polymer, for example in an amount of from about 0.05 to about 5%. Such agents are known generally for use in dentifrice, although not for this particular application, useful in the present disclosure are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as GANTREZ. e.g., AN 139 (M.W. 500,000), AN 119 (M.W. 250,000) and preferably S-97 Pharmaceutical Grade (M.W. 700,000) available from ISP Technologies, Inc., Bound Brook, N.J. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 Sikes et al.

### Water:

The oral compositions may comprise significant levels of water. Water employed in the preparation of commercial oral compositions should be deionized and free of organic impurities. The amount of water in the compositions includes the free water, which is added, plus that amount which is introduced with other materials.

### Humectants:

Within certain embodiments of the oral compositions, it is also desirable to incorporate a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In one embodiment, the principal humectant is glycerin, which may be present at levels of greater than 25%, e.g. about 25 to about 35% about 30%, with about 5% or less of other humectants.

### Other Optional Ingredients:

In addition to the above-described components, the embodiments disclosed herein can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

### Basic Amino Acids:

The basic amino acids which can be used in the compositions and methods of the present disclosure include not only naturally occurring basic amino acids, such as arginine, lysine, and histidine, but also any basic amino acids having a carboxyl group and an amino group in the molecule, which are water-soluble and provide an aqueous solution with a pH of 7 or greater.

Accordingly, basic amino acids include, but are not limited to, arginine, lysine, serine, citrullene, ornithine, creatine, histidine, diaminobutanoic acid, diaminoproprionic acid, salts thereof or combinations thereof. In a particular embodiment, the basic amino acids are selected from arginine, citrullene, and ornithine.

In certain embodiments, the basic amino acid is arginine, for example, L-arginine, or a salt thereof.

Suitable salts include salts known in the art to be pharmaceutically acceptable salts and are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

In certain embodiments, the basic amino acid is present in an amount corresponding to 0.1% to 15%, e.g., 0.1 weight % to 10 weight %, e.g., 0.1 to 5 wt%, e.g., 0.5 weight % to 3 weight % of the total composition weight, about e.g., 1%, 1.5%, 2%, 3%, 4%, 5%, or 8%, wherein the weight of the basic amino acid is calculated as free form.

### Surfactants:

The compositions disclosed herein, in some embodiments, may contain anionic surfactants, for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or, for example sodium laureth-2 sulfate (CH₃(CH₂)₁₀CH₂(OCH₂CH₂)₂OSO₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆-₃₀ alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

In another embodiment, cationic surfactants useful in the compositions disclosed herein can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di- isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants that can be used in the compositions can be broadly defined as compounds produced by condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition comprises a nonionic surfactant selected from polaxamers (e.g., POLAXAMER 407), POLYSORBATES (e.g., POLYSORBATE 20), polyoxyl hydrogenated castor oils (e.g., polyoxyl 40 hydrogenated castor oil), and mixtures thereof.

Illustrative amphoteric surfactants that can be used in the compositions may include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

The surfactant or mixtures of compatible surfactants can be present in the compositions in an amount of about 0.1% to about 5%, in another embodiment about 0.3% to about 3% and in another embodiment about 0.5% to about 2% by weight of the total composition.

### Flavoring Agents:

The oral care compositions may also include a flavoring agent. Flavoring agents which are used may include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1.5% by weight.

### Chelating and anti-calculus agents:

The oral care compositions also may include one or more chelating agents able to complex calcium found in the cell walls of the bacteria. Binding of this calcium weakens the bacterial cell wall and augments bacterial lysis.

Another group of agents suitable for use as chelating or anti-calculus agents are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide least 0.1 weight % pyrophosphate ions, e.g., 0.1 to 3 wt 5, e.g., 0.1 to 2 weight %, e.g., 0.1 to 1 wt%, e.g., 0.2 to 0.5 wt%. The pyrophosphates also contribute to preservation of the compositions by lowering water activity.

In preparing oral care compositions, a thickening material or agent may be utilized to provide a desirable consistency or to stabilize or enhance the performance of the formulation. In certain embodiments, the thickening agents are carboxyvinyl polymers, carrageenan, xanthan gum, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Silica may also be available as a thickening agent, e.g., synthetic amorphous silica. Colloidal magnesium aluminum silicate or finely divided silica can be used as component of the thickening composition to further improve the composition's texture. In certain embodiments, thickening agents in an amount of about 0.5% to about 5.0% by weight of the total composition are used. Thickeners may be present in an amount of from 1 weight % to 15 weight %, from 3 weight % to 10 weight %, 4 weight % to 9 weight %, from 5 weight % to 8 weight %, for example 5 weight %, 6 weight %, 7 weight %, or 8 weight %.

### Abrasives:

Natural calcium carbonate is found in rocks such as chalk, limestone, marble and travertine. It is also the principle component of egg shells and the shells of mollusks. The natural calcium carbonate abrasive is typically a finely ground limestone which may optionally be refined or partially refined to remove impurities. For use in the present composition, the material has an average particle size of less than 10 microns, e.g., 3-7 microns, e.g. about 5.5 microns. For example, a small particle silica may have an average particle size (D50) of 2.5 - 4.5 microns. Simply because natural calcium carbonate may contain a high proportion of relatively large particles of not carefully controlled, which may unacceptably increase the abrasivity, preferably no more than 0.01%, preferably no more than 0.004% by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred. An example of a commercially available product suitable for use in the present composition includes VICRON^{®} 25-11 FG from GMZ.

Precipitated calcium carbonate is generally made by calcining limestone, to make calcium oxide (lime), which can then be converted back to calcium carbonate by reaction with carbon dioxide in water. Precipitated calcium carbonate has a different crystal structure from natural calcium carbonate. It is generally more friable and more porous, thus having lower abrasivity and higher water absorption. The particles of the calcium carbonate are relatively small, e.g., having an average particle size of about 1 to about 5 microns, and e.g., no more than about 0.1 %, preferably no more than about 0.05% by weight of particles which would not pass through a 325 mesh. The particles may for example have a D50 of about 3 to about 6 microns, for example about 3.8 to about 4.9 microns, e.g., about 4.3 microns; a D50 of about 1 to about 4 microns, e.g. about 2.2 about 2.6 microns, e.g., about 2.4 microns, and a D10 of about 1 to about 2 microns, e.g., about 1.2 to about 1.4 microns, e.g. about 1.3 microns. The particles have relatively high water absorption, e.g., at least 25 g/l00g, e.g. about 30 to about 70 g/l00g. Examples of commercially available products suitable for use in the compositions and methods disclosed herein include, for example, CARBOLAG^{®} 15 Plus from Lagos Industria Quimica.

In certain embodiments the compositions disclosed herein may comprise additional abrasives, such as calcium-containing abrasives, for example calcium phosphate abrasive, e.g., tricalcium phosphate (Ca₃(PO₄)₂), hydroxyapatite (Ca₁₀(PO₄)₆(OH)₂), dicalcium phosphate dihydrate (CaHPO₄ · 2H₂O, also sometimes referred to herein as DiCal), calcium pyrophosphate, silica abrasives, sodium metaphosphate, potassium metaphosphate, aluminum silicate, calcined alumina, bentonite or other siliceous materials, or combinations thereof.

In certain embodiments, any silica suitable for oral care compositions may be used, such as precipitated silicas or silica gels. For example, the silica can also be small particle silica (e.g., SORBOSIL^{®} AC43 from PQ, Warrington, United Kingdom). The composition preferable contains from about 5 to about 20 weight % small particle silica, or for example about 10 to about 15 weight %, or for example about 5 weight %, about 10 wt%, about 15 weight % or about 20 weight % small particle silica.

In another embodiment, the abrasive may be high cleaning precipitated silica or high cleaning silica having a pellicle cleaning ratio (PCR) of greater than about 85 when tested at about 20% loading silica. Typically, high cleaning silica also has a mean particle size D₅₀ of from about 5 to about 15 µm and an oil absorption of from about 40 to about 120 cm³/100g silica. The cleaning efficacy of the precipitated silica is expressed using the pellicle cleaning ratio (PCR). This is typically measured at about 20% silica loading. The high cleaning silica preferably has a PCR value of greater than about 85. The efficacy of the precipitated silica can also be expressed with reference to its abrasive characteristic using the radioactive dentin abrasion (RDA). Ideally, RDA values for an oral composition should be below about 250 to protect tooth enamel/dentin. Methods of performing PCR and RDA are described in e.g., United States Patent Nos. 5,939,051 and 6,290,933 and "In Vitro Removal of Stain With Dentifrice", G. K. Stookey et al., J. Dental Research, Vol. 61, pages 1236-9, November 1982. Typically, the precipitated silica has a mean particle size D₅₀ of from about 5 to about 15 µm and an oil absorption of from about 40 to about 120 cm³/100g silica. Examples of precipitated silica having a mean particle size D₅₀ of from about 5 to about 15 µm and an oil absorption of from about 40 to about 120 cm³/100g silica include commercially available silicas such as ZEODENT^{®}103 and ZEODENT^{®}105 (Huber Silica Americas).

The composition preferable contains from about 3 to about 20 weight % high cleaning precipitated silica, or for example about 10 to about 15 weight %, or for example about 5 weight %, about 10 wt%, about 15 weight % or about 20 weight % high cleaning precipitated silica.

The composition may also comprise an abrasive silica having an acid pH in the composition. For example, prophy silica available from Grace, offered as SYLODENT^{™} , can be used. The acidic silica abrasive is included in the dentifrice components at a concentration of about 2 to about 35% by weight; about 3 to about 20 % by weight, about 3 to about 15% by weight, about 10 to about 15 % by weight. In certain embodiments, the acidic silica abrasive may be present in an amount between about 2 to about 7%. In other embodiments, it may be present in an amount from about 7 to about 15% by weight. Still on other embodiments, it may be present in an amount of from about 15 to about 30% by weight. For example, the acidic silica abrasive may be present in an amount selected from about 2 wt.%, about 3 wt.%, about 4% wt.%, about 5 wt.%, about 6 wt.%, about 7 wt.%, about 8 wt.%, about 9 wt.%, about 10 wt.%, about 11 wt.%, about 12 wt.%, about 13 wt.%, about 14 wt.%, about 15 wt.%, about 16 wt.%, about 17 wt.%, about 18 wt.%, about 19 wt.%, about 20 wt.%.

A commercially available acidic silica abrasive is SYLODENT 783 available from W. R. Grace & Company (Baltimore, MD). SYLODENT 783 has a pH of about 3.4 to about 4.2 when measured as an about 5% by weight slurry in water. In at least one embodiment, the silica material may have an average particle size of less than about 10 microns, e.g., about 3 to about 7 microns, e.g. about 5.5 microns.

In some embodiments, the compositions of the present disclosure contain a buffering agent. Examples of buffering agents include anhydrous carbonates such as sodium carbonate, sesquicarbonates, bicarbonates such as sodium bicarbonate, silicates, bisulfates, phosphates (e.g., monopotassium phosphate, dipotassium phosphate, tribasic sodium phosphate, sodium tripolyphosphate, phosphoric acid), citrates (e.g. citric acid, trisodium citrate dehydrate), pyrophosphates (sodium and potassium salts) and combinations thereof. The amount of buffering agent is sufficient to provide a pH of about 5 to about 9, preferable about 6 to about 8, and more preferable about 7, when the composition is dissolved in water, a mouthrinse base, or a toothpaste base. Typical amounts of buffering agent are about 5% to about 35%, in one embodiment about 10% to about 30%, in another embodiment about 15% to about 25%, by weight of the total composition.

Various other materials may be incorporated in the oral preparations of this disclosure such as whitening agents, preservatives, silicones, chlorophyll compounds and/or ammoniated material such as urea, diammonium phosphate, and mixtures thereof. These adjuvants, where present, are incorporated in the preparations in amounts which do not substantially adversely affect the properties and characteristics desired.

Any suitable flavoring or sweetening material may also be employed. Examples of suitable flavoring constituents are flavoring oils, e.g. oil of spearmint, peppermint, wintergreen, sassafras, clove, sage, eucalyptus, marjoram, cinnamon, lemon, and orange, and methyl salicylate. Suitable sweetening agents include sucrose, lactose, maltose, xylitol, sodium cyclamate, perillartine, AMP (aspartyl phenyl alanine, methyl ester), saccharine and the like. Suitably, flavor and sweetening agents may each or together comprise from about 0.1% to 5% more of the preparation. Moreover, flavoring oil is believed to aid the dissolving of the antibacterial agent, together with or even in the absence of surface-active agent.

In certain embodiments, the compositions or methods disclosed herein may make use of one or more antibacterial agents in addition to the zinc:usnate complex. Illustrative antibacterial agents may include, but are not limited to, benzalkonium chloride, benzethonium chloride, 5-bromo-5-nitro-1,3-dioxane, 2-bromo-2-nitropropane-1,3-diol, alkyl trimethyl ammonium bromide, N-(hydroxymethyl)-N-(1,3-dihydroxy methyl-2,5-dioxo-4-imidaxolidinyl-N-(hydroxy methyl)urea, 1-3-dimethyol-5,5-dimethyl hydantoin, formaldehyde, iodopropynl butyl carbamate, parabens, methylisothiazolinone, mixtures of methylisothiazolinone and methylchloroisothiazoline, mixtures of phenoxyethanol/butyl paraben/methyl paraben/propylparaben, 2-phenoxyethanol, tris-hydroxyethyl-hexahydrotriaz-ine, methylisothiazolinone, 5-chloro-2-methyl-4-isothiazolin-3-one, 1,2-dibromo-2,4-dicyanobutane, 1-(3-chloroalkyl)-3,5,7-triaza-azoniaadam-antane chloride, sodium benzoate, polyhexamethylene biguanide, alexidine, triclosan, parachlorometaxylenol, zinc pyrithione, essential oils (e.g., tea tree, eucaplyptus, thyme, etc.), silver and salts thereof, chlorhexidine and salts thereof, and the like, and combinations thereof. In a preferred embodiment, the antibacterial agent is phenoxyethanol (i.e., 2-phenoxyethanol).

Methods disclosed herein include applying to the oral cavity a safe and effective amount of the compositions described herein.

The following examples further describe and demonstrate illustrative embodiments within the scope of the present disclosure. The examples are given solely for illustration and are not to be construed as limitations of this disclosure as many variations are possible without departing from the invention which is defined in the claims. Various modifications of the disclosure in addition to those shown and described herein should be apparent to those skilled in the art and are intended to fall within the appended claims.

### Example 1 - Synthesis Method 1

Direct reaction of ZnO and usnic acid. To a scintillation vial was added about 21.2 mg of ZnO and about 183.2 mg of usnic acid (about 2.04 eq.) followed by about 4 mL of anhydrous EtOH. The capped mixture was stirred on a magnetic stirrer at about 80° C overnight. Sample was dry on next day morning, having a bright red-purple color. Mass Spectra of the material dissolved in EtOH confirmed Zn(Usn)₂. However, the red color was impurity. A purification attempt was performed by dissolving a portion of the crude product in EtOH, filtering and precipitating the product with DI water. The precipitate formed a colloidal solution that could not be filtered. Estimated yield of about less than 50%.

### Example 2 - Synthesis Method 2

Synthesis from zinc acetate and sodium usnate. To a small 4 mL vial was added about 31.9 mg of ZnO and about 47.4 mg of acetic acid (2.01 eq.) followed by about 0.5 mL of DI water. The solution was sonicated briefly to accelerate dissolution of ZnO to form a solution of zinc acetate (ZnAc₂). In a separate 20 mL scintillation vial, about 271.2mg (2.0 eq.) of usnic acid was added and stirred with a stirring bar. In a third vial was mixed about 63 mg of about 50% NaOH (2.0 eq.) with about about 3 mL of anhydrous EtOH. The NaOH was then added into the vial with usnic acid and stirred briefly to obtain a cloudy solution. About 1 mL of EtOH was used to rinse the NaOH vial to transfer all into the usnic acid vial. The ZnAc₂ was slowly added to the sodium usnate while stirring. The mixture was stirred overnight at room temperature (although the reaction was perhaps completed in about 1 to about 2 hours). The crude mixture was filtered to remove undissolved residue and product precipitated from filtrate by dilution with water. After filtration and air-drying, the product Zn(Usn)₂ was obtained as a light yellow powder, estimated yield of about 80 to about 92%. Analytical: Soluble in EtOH, insoluble in water. ¹H NMR (d6-DMSO, δ): 1.57 (CH₃). 1.93 (CH₃), 2.41 (CH₃), 2.56 (CH₃), 5.71 (CH), 12.35 (OH), 13.32 (OH). IR (neat, cm⁻¹): 1696, 1626, 1552, 1389, 1371, 1284, 1190, 1143, 1118, 1065, 1037, 843. Elemental analysis: 7.87% Zn.

The ¹H NMR analysis of the purified complex showed that only 2 OH groups per usnic moiety are present in the complex, while there were 3 OH groups in usnic acid. NMR diffusion coefficient experiment suggests a ratio of 2.5 between usnic acid and zinc. The IR analysis shows a shift of the carbonyl frequency in agreement with a metal binding. Elemental analysis of zinc is within about 10% error, which further confirms the stoichiometry of 1:2 identified by NMR.

### Example 3 - Synthesis Method 3

About 21.329 g of usnic acid and about 4.058 g of ZnO (0.805 eq.) were added to a 300-mL round bottom flask, followed by about 150 mL of anhydrous EtOH and then followed by slow addition of about 5.429 g of 50% NaOH/water (1.117 eq. to usnic acid) while stirring at room temperature (RT). After about 10 minutes, about 4.087 g of AcOH (1.003 eq. to NaOH) was added slowly over about 10 minutes and then stirred o/n. Stirring was stopped and after settling, the dark solution was decanted into about 800 mL of DI water while stirring to precipitate the product. The remaining EtOH slurry was centrifuged and the supernatant was added into the bulk beaker with water/product to precipitate. To drive more product precipitation, the suspension was cooled in the refrigerator at about 4°C overnight and the product was filtered, then washed with DI water and finally dried in vacuum oven to obtain about 23.4 g (100%) of product as a light yellow solid.

### Example 4 - Solubility Determination

Solubility of Zn:usnate complex was performed using standard techniques. Solubility of usnic acid in DI water at about 35 °C was reported to be about 6.3 ppm (see Jin et al., Solubility of (+)-Usnic Acid in Water, Ethanol, Acetone, Ethyl Acetate and n-Hexane, J Solution Chem., 2013, 42:1018-1027). The Zn:usnate complex showed a solubility of about 13.5 ppm in DI water at room temperature.

### Example 5 - Antibacterial Efficacy of Zinc:usnate Complexes on Oral Bacteria

Harvested bacteria (Actinomyces naeslundii, Lactobacillus casei, Streptococcus oralis, Fusobacterium nucleatum and Veilonella parvula) from a chemostat was centrifuged to about 1ml volume at about 15,000 rpm. Pelleted bacteria were re-suspended in the appropriate zinc:usnate complex treatment or in sterile media and ethanol. The tubes were then inverted 3 times and incubated at about 37 °C for about 1 hr. After incubation, about 1 mL of D/E neutralizing broth was added to each sample. Samples were then centrifuged at about 15,000 rpm for about 10 min to pellet bacteria. Supernatant was removed and pellet re-suspended in about 1 mL of 0.25x TSB. Samples were centrifuged at about 15,000rpm for about 10 min once more. Supernatant was removed and pellet re-suspended in about 1.5mL of 0.25x TSB. In a 96-well plate, about 100µL of samples were transferred in individual wells. Samples and standard curve were plated at least in triplicate. Once all samples and standard curve were transferred to the 96-well plate, about 100 µL of about 100 µg/mL solution of resazurin dye was added to each well. The plate was incubated at about 37 °C until a color change was observed at the 100% viability well of the standard curve. The plate was then read for fluorescence at 560 nm excitation/590 nm emission.

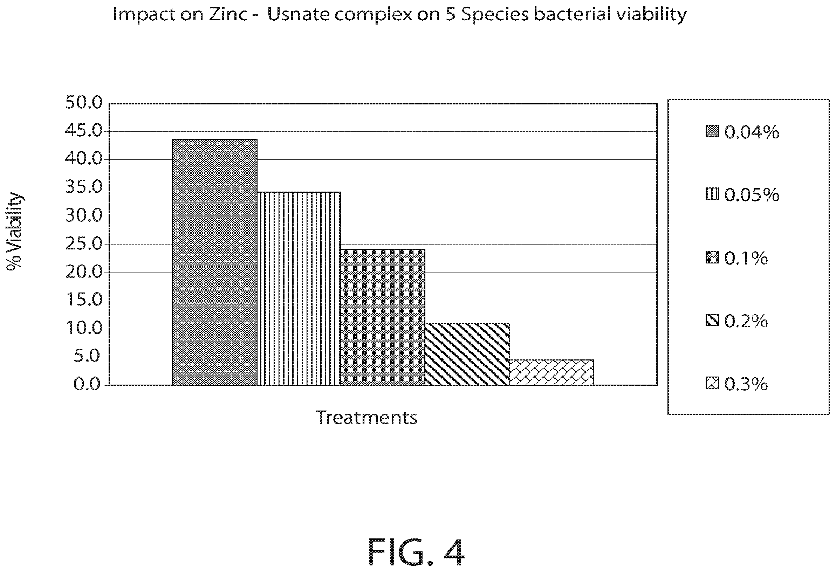

Results indicate that the zinc:usnate complex demonstrated a dose response based antibacterial effect (see Fig 4). The maximum reduction of the viable bacteria was achieved at about 0.3% of the concentration tested.

## Claims

1. An oral care composition comprising a Zn:usnic complex wherein said Zn:usnic acid complex has a 1:2 zinc to usnate molar ratio, wherein the oral care composition is selected from the group consisting of: a toothpaste, a dentifrice, a mouthwash, a mouth rinse, a topical oral gel, and a denture cleanser.

2. The composition according to claim 1, wherein said composition further comprises one or more agents selected from diluents, bicarbonate salts, pH modifying agents, surfactants, foam modulators, additional thickening agents, humectants, sweeteners, flavorants, pigments, antibacterial agents, anticaries agents, fluoride ion sources, anticalculus or tartar control agents, and mixtures thereof.

3. Oral care composition of any of the preceding claims for use in a method to improve oral health comprising applying an effective amount of said oral composition to the oral cavity of a subject in need thereof.

4. The oral care composition for use according to claim 3, wherein improving oral health is selected from one or more of the following;
a. reduce or inhibit formation of dental caries;
b. reduce, repair or inhibit early enamel lesions;
c. reduce or inhibit demineralization and promote remineralization of the teeth;
d. reduce hypersensitivity of the teeth;
e. reduce or inhibit gingivitis;
f. promote healing of sores or cuts in the mouth;
g. reduce levels of acid producing bacteria;
h. to increase relative levels of arginolytic bacteria;
i. inhibit microbial biofilm formation in the oral cavity;
j. raise and/or maintain plaque pH at levels of at least pH 5.5 following sugar challenge;
k. reduce plaque accumulation;
l. treat, relieve or reduce dry mouth;
m. whiten teeth;
n. enhance systemic health, including cardiovascular health;
o. reduce erosion of the teeth;
p. immunize the teeth against cariogenic bacteria and their effects;
q. clean the teeth and oral cavity;
r. reduce inflammation; and
s. increase anti-oxidant levels.

5. A method of preparing a Zn:usnate complex having a 1:2 zinc to usnate molar ratio, wherein preparation of said complex comprises the steps of:
a. mixing a zinc source and usnic acid;
b. adding ethanol;
c. mixing the solution at about 80° C; and
d. optionally isolating said complex.

6. A method of preparing a Zn:usnate complex having a 1:2 zinc to usnate molar ratio, wherein preparation of said complex comprises the steps of:
a. mixing a zinc source and acetic acid in water;
b. optionally sonicating the mixture;
c. in a separate container, mixing NaOH and ethanol;
d. mixing the NaOH and ethanol mixture with usnic acid;
e. mixing the solution of a. with the solution of d.; and
f. optionally isolating said complex.

7. A method of preparing a Zn:usnate complex having a 1:2 zinc to usnate molar ratio, wherein preparation of said complex comprises the steps of:
a. mixing a Zn source, usnic acid and ethanol;
b. adding acetic acid; and
c. optionally isolating said complex.

8. The method of any one of claims 5-7, wherein said Zn source is selected from zinc acetate and zinc oxide.

9. The method of claim 8, wherein said Zn source is zinc oxide.

10. The method of claim 8, wherein the Zn source is zinc acetate.

## Patentansprüche

1. Mundpflegezusammensetzung, umfassend einen Zn:Usninsäure-Komplex, wobei der Zn:Usninsäure-Komplex ein molares Verhältnis von Zink zu Usnat von 1:2 aufweist, wobei die Mundpflegezusammensetzung aus der Gruppe ausgewählt ist, bestehend aus: einer Zahnpasta, einem Zahnpflegemittel, einem Mundwasser, einer Mundspülung, einem topischen Mundgel und einem Reinigungsmittel für Zahnersatz.

2. Zusammensetzung nach Anspruch 1, wobei die Zusammensetzung ferner ein oder mehrere Mittel umfasst, ausgewählt aus Verdünnungsmitteln, Bicarbonatsalzen, pH-Modifikatoren, Tensiden, Schaumreglern, zusätzlichen Verdickungsmitteln, Feuchthaltemitteln, Süßungsmitteln, Aromastoffen, Pigmenten, antibakteriellen Mitteln, Karieshemmern, Fluoridionenquellen, Mitteln zur Zahnsteinverhinderung oder Zahnsteinkontrolle sowie Mischungen davon.

3. Mundpflegezusammensetzung nach einem der vorstehenden Ansprüche zur Verwendung in einem Verfahren zur Verbesserung der Mundgesundheit, umfassend das Auftragen einer wirksamen Menge der Mundpflegezusammensetzung in die Mundhöhle eines Patienten, der diese benötigt.

4. Mundpflegezusammensetzung zur Verwendung nach Anspruch 3, wobei die Verbesserung der Mundgesundheit aus einem oder mehreren der folgenden Punkte ausgewählt ist:
a. Verringerung oder Hemmung der Bildung von Zahnkaries;
b. Verringerung, Reparatur oder Hemmung früher Zahnschmelzläsionen;
c. Verringerung oder Hemmung der Demineralisierung und Förderung der Remineralisierung der Zähne;
d. Verringerung der Zahnüberempfindlichkeit;
e. Verringerung oder Hemmung von Gingivitis;
f. Förderung der Heilung von Wunden oder Schnitten im Mund;
g. Verringerung der Anzahl säureproduzierender Bakterien;
h. Erhöhung der relativen Anteil arginolytischer Bakterien;
i. Hemmung der Bildung mikrobieller Biofilme in der Mundhöhle;
j. Anhebung und/oder Aufrechterhaltung des pH-Werts des Zahnbelags nach einer Zuckerbelastung auf einem Wert von mindestens pH 5,5;
k. Verringerung der Ansammlung von Zahnbelag;
l. Behandelung, Linderung oder Verringerung von Mundtrockenheit;
m. Aufhellung der Zähne;
n. Verbesserung der systemischen Gesundheit, einschließlich der Herz-Kreislauf-Gesundheit;
o. Verringerung der Erosion der Zähne;
p. Immunisierung der Zähne gegen kariogene Bakterien und deren Auswirkungen;
q. Reinigung der Zähne und der Mundhöhle;
r. Linderung von Entzündungen; und
s. Erhöhung der Menge an Antioxidantien.

5. Verfahren zur Herstellung eines Zn:Usnat-Komplexes mit einem molaren Verhältnis von Zink zu Usnat von 1:2, wobei die Herstellung des Komplexes die folgenden Schritte umfasst:
a. Mischen einer Zinkquelle und von Usninsäure;
b. Zugabe von Ethanol;
c. Mischen der Lösung bei etwa 80 °C; und
d. optional, Isolieren des Komplexes.

6. Verfahren zur Herstellung eines Zn:Usnat-Komplexes mit einem Molverhältnis von Zink zu Usnat von 1:2, wobei die Herstellung des Komplexes die folgenden Schritte umfasst:
a. Mischen einer Zinkquelle und von Essigsäure in Wasser;
b. optional, Beschallen der Mischung;
c. Mischen von NaOH und Ethanol in einem separaten Behälter;
d. Mischen der NaOH- und Ethanol-Mischung mit Usninsäure;
e. Mischen der Lösung aus a. mit der Lösung aus d.; und
f. optional, Isolieren des Komplexes.

7. Verfahren zur Herstellung eines Zn:Usnat-Komplexes mit einem Molverhältnis von Zink zu Usnat von 1:2, wobei die Herstellung des Komplexes die folgenden Schritte umfasst:
a. Mischen einer Zn-Quelle, Usninsäure und Ethanol;
b. Zugabe von Essigsäure; und
c. optional, Isolieren des Komplexes.

8. Verfahren nach einem der Ansprüche 5 bis 7, wobei die Zn-Quelle ausgewählt ist aus Zinkacetat und Zinkoxid.

9. Verfahren nach Anspruch 8, wobei die Zn-Quelle Zinkoxid ist.

10. Verfahren nach Anspruch 8, wobei die Zn-Quelle Zinkacetat ist.

## Revendications

1. Composition de soins bucco-dentaires comprenant un complexe Zn:acide usnique, dans laquelle ledit complexe Zn:acide usnique présente un rapport molaire zinc/usnate de 1:2, et dans laquelle la composition de soins bucco-dentaires est choisie dans le groupe constitué par : une pâte dentifrice, un dentifrice, un bain de bouche, une solution de rinçage buccal, un gel buccal topique et un produit de nettoyage pour prothèses dentaires.

2. Composition selon la revendication 1, dans laquelle ladite composition comprend en outre un ou plusieurs agents choisis parmi les diluants, les sels de bicarbonate, les agents modificateurs de pH, les tensioactifs, les modificateurs de mousse, les agents épaississants supplémentaires, les humectants, les édulcorants, les agents aromatisants, les pigments, les agents antibactériens, les agents anticaries, les sources d'ions fluorure, les agents antitartre et les mélanges de ceux-ci.

3. Composition de soins bucco-dentaires selon l'une quelconque des revendications précédentes, pour une utilisation dans un procédé visant à améliorer la santé bucco-dentaire, comprenant l'application d'une quantité efficace de ladite composition bucco-dentaire à la cavité buccale d'un sujet en ayant besoin.

4. Composition de soins bucco-dentaires pour une utilisation selon la revendication 3, dans laquelle l'amélioration de la santé bucco-dentaire est choisie parmi un ou plusieurs des éléments suivants :
a. réduire ou inhiber la formation de caries dentaires ;
b. réduire, réparer ou inhiber les lésions précoces de l'émail ;
c. réduire ou inhiber la déminéralisation et favoriser la reminéralisation des dents ;
d. réduire l'hypersensibilité dentaire ;
e. réduire ou inhiber la gingivite ;
f. favoriser la cicatrisation des plaies ou coupures dans la bouche ;
g. réduire les taux de bactéries productrices d'acide ;
h. augmenter les taux relatifs de bactéries arginolytiques ;
i. inhiber la formation de biofilm microbien dans la cavité buccale ;
j. élever et/ou maintenir le pH de la plaque à un niveau d'au moins 5,5 après une exposition aux sucres ;
k. réduire l'accumulation de plaque ;
l. traiter, soulager ou réduire la sécheresse de la bouche ;
m. blanchir les dents ;
n. améliorer la santé systémique, y compris la santé cardiovasculaire ;
o. réduire l'érosion dentaire ;
p. immuniser les dents contre les bactéries cariogènes et leurs effets ;
q. nettoyer les dents et la cavité buccale ;
r. réduire l'inflammation ; et
s. augmenter les taux d'antioxydants.

5. Procédé de préparation d'un complexe Zn:usnate présentant un rapport molaire zinc/usnate de 1:2, dans lequel la préparation dudit complexe comprend les étapes de :
a. mélange d'une source de zinc et d'acide usnique ;
b. ajout d'éthanol ;
c. mélange de la solution à environ 80 °C ; et
d. éventuellement isolement dudit complexe.

6. Procédé de préparation d'un complexe Zn:usnate présentant un rapport molaire zinc/usnate de 1:2, dans lequel la préparation dudit complexe comprend les étapes de :
a. mélange d'une source de zinc et d'acide acétique dans de l'eau ;
b. éventuellement soumission du mélange à une sonication ;
c. dans un récipient distinct, mélange de NaOH et d'éthanol ;
d. mélange du mélange de NaOH et d'éthanol avec de l'acide usnique ;
e. mélange de la solution de a. avec la solution de d. ; et
f. éventuellement isolement dudit complexe.

7. Procédé de préparation d'un complexe Zn:usnate présentant un rapport molaire zinc/usnate de 1:2, dans lequel la préparation dudit complexe comprend les étapes de :
a. mélange d'une source de Zn, d'acide usnique et d'éthanol ;
b. ajout d'acide acétique ; et
c. éventuellement isolement dudit complexe.

8. Procédé selon l'une quelconque des revendications 5 à 7, dans lequel ladite source de Zn est choisie parmi l'acétate de zinc et l'oxyde de zinc.

9. Procédé selon la revendication 8, dans lequel ladite source de Zn est l'oxyde de zinc.

10. Procédé selon la revendication 8, dans lequel la source de Zn est l'acétate de zinc.
